# EUROPEAN PATENT SPECIFICATION

(11) **EP 2 601 844 B1**
(45) Date of publication and mention of the grant of the patent: **21.10.2015**
(21) Application number: 11191897.5
(22) Date of filing: 05.12.2011
(51) Int. Cl.: A23F 3/18, A23F 5/20, A23L 1/30, A61K 31/4425

(54) **Method of producing an extract enriched with trigonelline (TRIG) and/or chlorogenic acids (CQA)**
Verfahren zur Herstellung eines mit Trigonellin (TRIG) und/oder Chlorogensäuren (CQA) angereichertem Extrakt
Procédé pour produire un extrait enrichi en trigonelline (TRIG) et/ou acides chlorogéniques (CQA)

(43) Date of publication of application: 12.06.2013
(73) Proprietor: Plantextrakt GmbH & Co. KG, 91487 Vestenbergsgreuth (DE)
(72) Inventor: Bonnländer, Dr. Bernd, 90571 Schwaig (DE); Kriesl, Erwin, 90579 Langenzenn (DE); Perera, Harsha, 90443 Nürnberg (DE)
(74) Representative: Rau, Schneck & Hübner Patentanwälte Rechtsanwälte PartGmbB

(56) References cited:
- DE-A1- 1 692 249
- DE-A1- 3 915 535
- DE-A1- 4 316 654
- JP-A- 2011 083 282
- US-A- 4 160 042
- US-A1- 2007 003 683

## Description

The invention refers to a method of producing an extract enriched with trigonelline (TRIG) and/or chlorogenic acids (CQA).

The field of the invention refers to functional plant extracts of special species like raw coffee and mate. In this connection attention is to be drawn to the fact that coffee is known as a major source of antioxidants. Its positive effects on health are largely documented in actual scientific literature. In raw coffee chlorogenic acids (CQA) are known as most abundant polyphenols which are partly destroyed during roasting. Nevertheless coffee as beverage is one of the main contributors to the intake of antioxidants and due to the remaining chlorogenic acids. Hereunder a family of esters formed between transcinnamic acid (caffeoylquinic acid) and quinic acid are classically summarized:
- CQA (caffeoylquinic acids)
- pCoCQA (p-coumaroylcaffeoylquinic acids)
- FQA (feruloylquinic acids)
- diCQA (dicaffeoylquinic acids) and
- CFQA (caffeoylferuloylquinic acid)

In broader range all structurally similar compounds as listed in the following formula overview occur in nature.

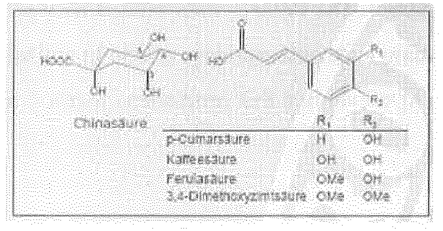

Another compound especially present in coffee is trigonelline, an alkaloid which is correlated to other positive health effects of coffee, especially after it has been transformed during the roasting process.

The currently known prior art can roughly be outlined as follows:
In literature, e.g. in the so-called Farah overview, which is Viviane Marques, Adriana Farah, "Chlorogenic acids and related compounds in medicinal plants and infusions" in Food Chemistry 113 (2009), pages 1370 - 1376, different plant materials were evaluated for the content of CQAs, the so called secondary plant metabolites. Products are on the market containing up to 45 % of this CQAs enriched by solvent extraction either from Europe or India.

The health benefits described are slimming or antioxidant properties. The slimming effect is attributed to a reduced utilization of carbohydrates by enzyme inactivation (amylases).

The trigonelline in coffee is the precursor of NMP (n-methylium pyridine) which is currently reported as a diabetes active principle in WO 2010/055170 A1.

WO 2010/054818 A1 discloses a method of preparation a NMP-containing extract starting with roasting trigonelline containing plant material (as e.g. coffee,). The roasted trigonelline-containing organic material is then treated with water to obtain an aqueous extract. The latter is then treated with polyamide and subjected to cation exchange chromatography to obtain a NMP-containing extract. Due to the use of polyamide this production method is not exclusively natural.

Trigonelline is further known in cosmetic applications, as disclosed in DE 3915535 A1.

From FR 2 734 479 A1 a process is known for the preparation of a green coffee extract in which the raw plant material is subjected to organic solvents like ethanol, from which traces may remain in the product, what is undesired for offering residue-free, certified (organic) products. Further on in the described process the decaffeinated raw coffee material is ground. This shows considerable disadvantages compared to processes in which the whole green coffee bean is extracted. Technical problems are e.g. the separation solid-liquid and enrichment, namely the distribution of CGA in the coffee bean.

The most relevant prior art documents JP 2011083282 A and US 2007/003683 A1 refer to extraction methods which are based on roasted coffee beans or processed tea leaves following tea manufacturing.

According to the first document the main aspect is the removal of raw bean smell from a coffee extract product by deodorizing roasted coffee beans. This is done by steam distillation and extracting the resultant product with water, ethanol or a mixture thereof.

The second document refers to a method of extracting volatile components from tasty material like roasted coffee beans or tea leaves following tea manufacturing. Preferably super heated steam is used for processing the tasty material.

According to US 4 160 042 A an aqueous extract of a vegetable material is treated with a view to reducing its caffeine and/or chlorogenic acid content. The treatment is with a solid ligneous adsorbent of vegetable origin in a divided form at a temperature from 0°C to 100°C.

Starting from the prior art problems it is an object of the invention to present a method of producing an extract enriched with trigonelline (TRIG) and/or chlorogenic acids (CQA) in an all natural way, only by using water and steam.

This object is achieved by the method presented in claim 1 referring to following method steps:
(a) providing an amount of the TRIG and/or CQA containing organic plant material in a container, said at least one organic plant material are green beans of Coffea arabica and/or Coffea canephoris or mate leaves,
(b) treating said amount of TRIG and/or CQA containing organic plant material with steam for a defined vaporisation period,
(c) exposing the TRIG and/or CQA containing organic plant material steamed in step (b) to hot water for at least two times for extraction, and
(d) removing said extract, in each time gained in step (c), enriched with trigonelline (TRIG) and/or chlorogenic acid (CQA) from the residue.

The method according to the invention thus offers the possibility to supply such an extract as organic certified material, when starting from such raw plant material. The present inventor has surprisingly found out, that merely by a fractionated extraction by hot water after steam treatment, it is possible to obtain a trigonelline and CQA rich extract of different plant materials like coffee beans, mate and other Rubiaceae plant material or combinations thereof.

The vaporisation in step (b) may be performed by saturated, preferably dry steam for at least 3 min to 15 min, more preferably 5 min to 12 min, most preferably 10 min, wherein the steam pressure may be 4 bar to 6 bar, preferably 5 bar.

According to another preferred embodiment of the invention the temperature of the hot water used in step(s) (c) is 75°C to 95°C, preferably 80°C to 90°C, wherein the TRIG and/or CQA containing organic plant material may further be exposed to the hot water for a time period of 1 min to 20 min, preferably 10 min.

The process of maceration, i.e. subjecting the plant material to the hot water with an according extraction of the interesting substances TRIG and/or CQA and the subsequent removal of the extract maybe repeated successively for up to 15 times, preferably 5 to 10 times depending on the yield of extraction.

According to a further preferred embodiment of the invention the TRIG and/or CQA containing organic plant material before used in step (a) is subjected to a liquid carbon dioxide extraction, preferably to a supercritical carbon dioxide extraction. By this step a product with very low caffeine content can be obtained.

Further on it is possible that the extract obtained in step(s) (d) is concentrated, preferably by RO (reverse osmosis), thin film evaporation or any other gentle concentration technique to obtain a highly viscose, concentrated product. Thus the product can easily be pasteurized before being filled in adequate containers like plastic bags.

Another preferred alternative for the processing of the extract as to reasonable marketing conditions is spray or freeze drying the aqueous extract to a powdered extract product.

The extract enriched with trigonelline (TRIG) and/or chlorogenic acids (CQA), produced by the above outlined method may be used for direct enrichment of all natural products with natural antioxidants and/or as completely natural source of trigonelline and/or for naturally colouring food products. The extract can give a pleasant, all natural green colour to any food product.

Summing up the extract produced according to the invention can be used as a food, to fortify other food products or as supplement as it is. Further on it can be used as source of high trigonelline content for use as it is or to produce NMP if applied to coffee before roasting. Opposed to US 2010/0112098 A1 where the application for weight reduction is described, this invention is directed to a process for producing an all natural extract, showing coloring properties and an enhanced content of CQA / TRIG as functional active ingredients

The invention will now be explained more detailed based on following:

### Examples:

### 1. Basic process technique

Raw green coffee beans or green mate leaves are steam treated to open the cellular structure of the leave or seed material. This is referable done with saturated steam of 2 bar for 10 min in an extraction vessel - step (a).

In a second step hot water preferably of 90° C is added to this swollen material until covered. After a time of 1-20 minutes, preferably 10 min the extract is removed and the plant material is again covered with hot water of 85° C. The maceration and extraction is repeated 5 times to reach a total extraction yield of 12% - steps (b).

After each extraction step (b) the extract enriched with trigonelline (TRIG) and/or chlorogenic acid (CQA) is removed from the residue by filtering or centrifugation - step (c).

The first fraction containing higher amounts of CQA and TRIG is kept apart and a ratio of these two physiologically active substances can be blended by mixing different fractions in different ratios.

The following table 1 shows the content of 5-CQA, total CQA and TRIG against fractions of coffee bean extraction. It is an expression of the natural variability (concerning ingredients and structures) which has influence on the extraction process and the contents in the extract.

**Table 1**

| **FRACTION NO** | **SUM OF CQA/FQA/COQA = TCQA [DRY MATTER]** | | **5-CQA** | **SUM OF TRIG** |
|---|---|---|---|---|
| | **Coffee 1** | **Coffee 2** | **[dm]** | **[dm]** |
| **1** | 45 % | 24 % | 10 % | 4,7 % |
| **2** | 54 % | 35 % | 13 % | 4,8 % |
| **3** | 55 % | 37 % | 14 % | 4,5 % |
| **4** | 63 % | 46 % | 19 % | 4,0 % |
| **5** | 62 % | 45 % | 19 % | 3,5 % |

Additionally to the above-mentioned process steps the coffee beans are decaffeinated by supercritical or liquid CO₂ before said process, which is sufficiently known to the skilled persons and does not need any further explanation here.

Further on the beans or leaves may be shortly heated to 150°C for 5 minutes before said process, which light roasting does not significantly impair the profile of the product. Otherwise TRIG/CQA may even be destroyed during the full roasting, see Clarke, R. J., Vitzthum, O. G. (Ed.), "Coffee-Recent Developments", Blackwell Science, 2001.

### 2. Extraction of Arabica coffee beans

1 kg of graded and sieved Coffea arabica beans of Brasil origin (unwashed) were filled into a glass extraction cylinder and treated with saturated but dry steam for 5 minutes.

The swollen beans were extracted by 5 1 of demineralized water of 90°C two times gaining a yield of 20 % extracted matter with a tCQA content of 15%, 1/3 of which was CQA.

### 3. Extraction of decaffeinated Robusta beans

3 t Coffea canephora (Pierre ex Froehn) beans from Uganda were decaffeinated by a process of CO₂. 2.6 kg of these bean were treated by saturated steam in an extraction cylinder and consecutively extracted by portions of 2,61 water of 90°C for 10 times. A total of 45% CQAs could be reached with a ratio of 6 or 5:1 (3-CQA/5-CQA or 4-CQA/5CQA)

### 4. Extraction of mate leaves

0,5 kg of green mate leaves were extracted by 11 of water with temperature of 85°C for 5 minutes after dry saturated steam treatment of 1 minute with 0,5 bar. The extraction was repeated 5 times and the fractions were analyzed for tCQA. The result (percent by weight related to dry matter) is presented in the following table 2.

**Table 2**

| FRACTION | TCQA [% DM] | 5-CQA [% DM] |
|---|---|---|
| 1 | 26 | 8 |
| 2 | 28 | 8 |
| 3 | 32 | 9 |
| 4 | 26 | 7 |
| 5 | 32 | 8 |
| 6 | 31 | 7 |

### 5. Control extraction of Trigonella

1 kg of Trigonella foenum-graecum L. seeds were extracted but showed max content of 2 % of trigonelline. From the plant Trigonella Foenumgraecum L. trigonelline was isolated for the first time - the name trigonelline has its origin from this plant. But the trigonelline contents from this plant are too little for an economic extraction.

## Claims

1. Method of producing an extract enriched with trigonelline (TRIG) and/or chlorogenic acids (CQA) from a TRIG and/or CQA containing organic plant material like Rubiacea plants or mate plants, the method comprising the steps of:
(a) providing an amount of the TRIG and/or CQA containing organic plant material in a container, said at least one organic plant material being selected of the group of green beans of Coffea arabica and Coffea canephoris, and mate leaves,
(b) treating said amount of TRIG and/or CQA containing organic plant material with steam for a defined vaporisation period,
(c) exposing the TRIG and/or CQA containing organic plant material steamed in step (b) to hot water for at least two times for extraction, and
(d) removing said extract, in each time gained in step (c), enriched with trigonelline (TRIG) and/or chlorogenic acid (CQA) from the residue.

2. Method according to claim 1, wherein the vaporisation in step (b) is performed by saturated, preferably dry steam for at least 3 min to 15 min, more preferably 5 min to 12 min, most preferably 10 min.

3. Method according to claim 2, wherein the steam pressure in step (b) is 4 bar to 6 bar, preferably 5 bar.

4. Method of claim 2 or 3, wherein the vaporisation in step (b) is performed in an extraction vessel.

5. Method according to one of the preceding claims, wherein the temperature of the hot water used in step(s) (c) is 75°C to 95°C, preferably 80°C to 90°C.

6. Method according to claim 5, wherein the TRIG and/or CQA containing organic plant material is exposed to the hot water for a time period of 1 min to 20 min, preferably 10 min.

7. Method according to claim 5 or 6, wherein the step (c) in combination with step (d) is repeated successively for up to 15 times, preferably 5 to 10 times.

8. Method according to one of the preceding claims, wherein the TRIG and/or CQA containing organic plant material before used in step (a) is subjected to a liquid carbon dioxide extraction, preferably to a supercritical carbon dioxide extraction.

9. Method according to one of the preceding claims, wherein the extract obtained in step(s) (d) is concentrated, preferably by reverse osmosis or thin film evaporation to obtain a highly viscose, concentrated product.

10. Method according to one of the preceding claims, wherein the extract obtained in step(s) (d) or the highly viscose, concentrated product is subjected to spary or freeze drying to obtain a powdered extract product.

## Patentansprüche

1. Verfahren zur Herstellung eines mit Trigonellin (TRIG) und/oder Chlorogensäuren (CQA) angereicherten Extrakts aus einem TRIG und/oder CQA enthaltenden biologischen Pflanzenmaterial wie z.B. Rubiacea-Pflanzen oder Matepflanzen, wobei das Verfahren die folgenden Schritte umfasst:
(a) Bereitstellen einer Menge des TRIG und/oder CQA enthaltenden biologischen Pflanzenmaterials in einem Behälter, wobei das zumindest eine organische Pflanzenmaterial ausgewählt ist aus der Gruppe umfassend die grünen Bohnen der Coffea arabica- und der Coffea canephoris-Pflanze sowie Mateblätter,
(b) Behandeln der Menge des TRIG und/oder CQA enthaltenden biologischen Pflanzenmaterials mit Dampf in einem bestimmten Bedampfungszeitraum,
(c) mindestens zweimaliges Beaufschlagen des in Schritt (b) bedampften, TRIG und/oder CQA enthaltenden biologischen Materials mit Wasser zum Zwecke einer Extraktion, und
(d) Entfernen des jeweils in Schritt (c) gewonnenen, mit Trigonnellin (TRIG) und/oder Chlorogensäure (CQA) angereicherten Extraktes aus den Rückständen.

2. Verfahren gemäß Anspruch 1, wobei die Bedampfung in Schritt (b) mit Hilfe von gesättigtem, vorzugsweise trockenem Dampf für einen Zeitraum von mindestens 3 min bis 15 min, bevorzugter 5 min bis 12 min, am bevorzugtesten 10 min, durchgeführt wird.

3. Verfahren gemäß Anspruch 2, wobei der Dampfdruck in Schritt (b) 4 bar bis 6 bar, vorzugsweise 5 bar, beträgt.

4. Verfahren gemäß Anspruch 2 oder 3, wobei die Bedampfung in Schritt (b) in einem Extraktionsgefäß durchgeführt wird.

5. Verfahren gemäß einem der vorherigen Ansprüche, wobei die Temperatur des in dem (den) Schritt(en) (c) verwendeten heißen Wassers bei 75°C bis 95°C, vorzugsweise 80°C bis 90°C, liegt.

6. Verfahren gemäß Anspruch 5, wobei das TRIG und/oder CQA enthaltende biologische Pflanzenmaterial für einen Zeitraum von 1 min bis 20 min, vorzugsweise 10 min, mit dem heißen Wasser beaufschlagt wird.

7. Verfahren gemäß Anspruch 5 oder 6, wobei der Schritt (c) in Verbindung mit Schritt (d) nacheinander bis zu 15 Mal, vorzugsweise 5 bis 10 Mal, wiederholt wird.

8. Verfahren gemäß einem der vorherigen Ansprüche, wobei das TRIG und/oder CQA enthaltende biologische Pflanzenmaterial vor Verwendung in Schritt (a) einer Extraktion mit flüssigem Kohlendioxid, vorzugsweise einer Extraktion mit überkritischem Kohlendioxid, unterzogen wird.

9. Verfahren gemäß einem der vorherigen Ansprüche, wobei der in dem (den) Schritt(en) (d) gewonnene Extrakt zur Gewinnung eines hochviskosen konzentrierten Produkts vorzugsweise mittels Umkehrosmose oder Dünnschichtverdampfung konzentriert wird.

10. Verfahren gemäß einem der vorherigen Ansprüche, wobei der in dem (den) Schritt(en) (d) gewonnene Extrakt oder das hochviskose konzentrierte Produkt zur Gewinnung eines pulverförmigen Extrakt-Produkts einem Sprühtrocknungs- oder Gefriertrocknungsprozess unterzogen wird.

## Revendications

1. Procédé de production d'un extrait enrichi en trigonelline (TRIG) et/ou en acides chlorogéniques (CQA) provenant d'un matériel végétal organique contenant TRIG et/ou CQA telle que des plantes appartenant aux Rubiacées ou des plantes de maté, le procédé comprenant les étapes qui consistent :
(a) à fournir une quantité du matériel végétal organique contenant TRIG et/ou CQA dans un récipient, ledit au moins un matériel végétal organique étant choisi dans le groupe des haricots verts de Coffea arabica et de Coffea canephoris, et de feuilles de maté,
(b) à traiter ladite quantité de matériel végétal organique contenant TRIG et/ou CQA avec de la vapeur pendant une période de vaporisation définie,
(c) à exposer le matériel végétal organique contenant TRIG et/ou CQA traité à la vapeur dans l'étape (b) à de l'eau chaude au moins deux fois pour l'extraction, et
(d) à retirer ledit extrait, à chaque fois obtenu dans l'étape (c), enrichi en trigonelline (TRIG) et/ou en acide chlorogénique (CQA) du résidu.

2. Procédé selon la revendication 1, dans lequel la vaporisation dans l'étape (b) est effectuée par de la vapeur saturée, de préférence sèche pendant au moins 3 min à 15 min, plus préférablement pendant 5 min à 12 min, le plus préférablement pendant 10 min.

3. Procédé selon la revendication 2, dans lequel la pression de vapeur dans l'étape (b) est comprise entre 4 bars et 6 bars, de préférence de 5 bars.

4. Procédé de la revendication 2 ou 3, dans lequel la vaporisation dans l'étape (b) est effectuée dans une cuve d'extraction.

5. Procédé selon l'une des revendications précédentes, dans lequel la température de l'eau chaude utilisée dans l'étape/les étapes (c) est comprise entre 75°C et 95°C, de préférence entre 80°C et 90°C.

6. Procédé selon la revendication 5, dans lequel le matériel végétal organique contenant TRIG et/ou CQA est exposé à l'eau chaude pendant une durée allant de 1 min à 20 min, de préférence de 10 min.

7. Procédé selon la revendication 5 ou 6, dans lequel l'étape (c) en combinaison avec l'étape (d) est répétée successivement jusqu'à 15 fois, de préférence 5 à 10 fois.

8. Procédé selon l'une des revendications précédentes, dans lequel le matériel végétal organique contenant TRIG et/ou CQA avant d'être utilisé dans l'étape (a) est soumis à une extraction au dioxyde de carbone liquide, de préférence à une extraction au dioxyde de carbone supercritique.

9. Procédé selon l'une des revendications précédentes, dans lequel l'extrait obtenu dans l'étape/les étapes (d) est concentré, de préférence par osmose inverse ou évaporation en couche mince afin d'obtenir un produit concentré, à viscosité élevée.

10. Procédé selon l'une des revendications précédentes, dans lequel l'extrait obtenu dans l'étape/les étapes (d) ou le produit concentré, à viscosité élevée est soumis à un séchage par pulvérisation ou à une lyophilisation pour obtenir un produit extrait en poudre.
